# EUROPÄISCHE PATENTSCHRIFT

(11) **EP 2 060 246 B1**
(45) Veröffentlichungstag und Bekanntmachung des Hinweises auf die Patenterteilung: **06.01.2010**
(21) Anmeldenummer: 08019827.8
(22) Anmeldetag: 13.11.2008
(51) Int. Cl.: A61F 9/02

(54) **Skibrille**
Ski goggles
Lunettes de ski

(30) Priorität: 13.11.2007 EP 07021954
(43) Veröffentlichungstag der Anmeldung: 20.05.2009
(73) Patentinhaber: ALPINA SPORTS GmbH, 86316 Friedberg (DE)
(72) Erfinder: Grau, Werner, 86316 Friedberg/Haberskirch (DE)
(74) Vertreter: Bauerschmidt, Peter

(56) Entgegenhaltungen:
- WO-A-00/69296
- WO-A-2005/107667
- US-A- 3 395 406
- US-A- 4 435 852
- US-A- 5 652 965

## Beschreibung

Die Erfindung richtet sich auf eine Skibrille, umfassend einen Rahmen und eine daran angeordnete Sichtscheibe sowie ein Halteband und Lufteinlassöffnungen im Rahmen.

Der Rahmen ist so ausgestaltet, dass die Scheibe im Abstand zu den Augen des Benutzers gehalten wird, d.h. zwischen der Innenseite der Sichtscheibe und dem Gesicht ist ein abgeschlossener Luftraum. Dies führt dazu, dass dieser Raum sich erwärmt und aufgrund der Transpiration des Benutzers eine höhere Feuchtigkeit aufweist als die Umgebung, was wiederum zu einem Beschlagen der Innenseite der Sichtscheibe führt.

Einem solchen Beschlagen kann man durch eine entsprechende Beschichtung einerseits und durch Lufteinlassöffnungen andererseits entgegenwirken, welche dafür sorgen, dass ein Luftaustausch zwischen dem feuchteren, wärmeren Innenraum und der Umgebungsluft stattfindet. Eine Skibrille mit Belüftungsöffnungen ist beispielsweise aus der US 3,395,406 bekannt.

Hiervon ausgehend liegt der Erfindung die Aufgabe zugrunde, durch Optimierung dieses Luftaustausches dem Beschlagen der Scheibeninnenseite entgegenzuwirken.

Diese Aufgabe wird erfindungsgemäß dadurch gelöst, dass oberhalb der Scheibe ein die Vorderseite der Scheibe überragender Schild angeordnet ist, welches Lufteinlassöffnungen aufweist, wobei der Schild im Abstand zum Rahmen derart angeordnet ist, dass Luft durch die Lufteinlassöffnungen und unterhalb des Schildes einströmen kann.

Insbesondere ist mit Vorteil vorgesehen, dass im Mittelbereich die einströmende Luft durch Durchlassöffnungen des Rahmens in den Bereich zwischen der Hinterseite der Scheibe und der Stirn des Benutzers gelenkt wird.

In den Randbereichen ist demgegenüber günstigerweise vorgesehen, dass die durch die Einlassöffnungen einströmende Luft oberhalb von Durchlassöffnungen im Rahmen zum Bereich hinter der Scheibe entlanggeführt und durch Auslassöffnungen an der Oberseite des Rahmens wieder herausgeführt wird.

Durch diesen Luftstrom wird im Bereich hinter der Scheibe ein Unterdruck erzeugt, so dass aus diesem Bereich feuchte, warme Luft eingesaugt und nach außen mit herausgeführt wird.

Besonders vorteilhafte Strömungsverhältnisse erhält man dadurch, dass das Schild mit der Scheibe einen Winkel von < als 90° einschließt.

Nachfolgend wird die Erfindung anhand eines bevorzugten Ausführungsbeispiels in Verbindung mit der Zeichnung näher erläutert. Dabei zeigen:

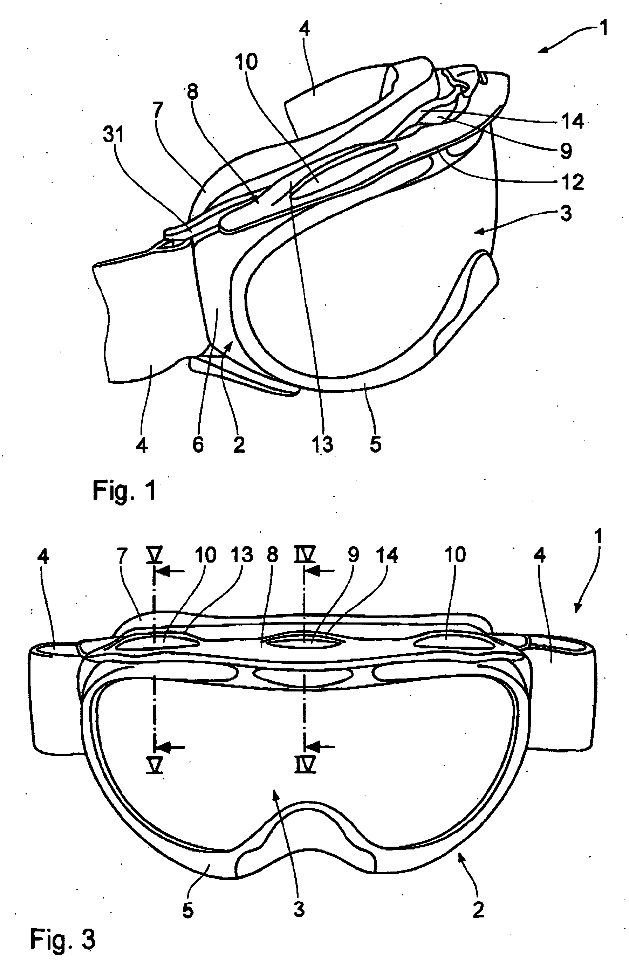

Fig. 1 eine perspektivische Ansicht einer erfindungsgemäßen Skibrille,
Fig. 2 eine Aufsicht der Skibrille,
Fig. 3 eine Ansicht von vorne,
Fig. 4 einen Schnitt längs der Linie IV-IV in Fig. 3 und
Fig. 5 einen Schnitt längs der Linie V-V in Fig. 3.

Eine in der Zeichnung dargestellte Skibrille 1 umfasst einen Rahmen 2 , an dem eine Sichtscheibe 3 angeordnet ist, und der mit einem Halteband 4 versehen ist. Das Halteband 4 ist mittels eines Kopfbandbügels 31 mit dem Rahmen 2 verbunden. Der Kopfbandbügel 31 ist in einem seitlichen, peripheren Bereich am Rahmen 2 angeordnet und erstreckt sich entlang der Oberkante der Skibrille 1. Der Kopfbandbügel 31 kann in einer besonders ansprechenden Ausführungsform in eine Kontrastfarbe zum Rahmen 2 ausgebildet sein. Es ist vorzugsweise aus Kunststoff und/oder Metall.

Der Rahmen 2 weist einen die Scheibe 3 umgreifenden Rahmenabschnitt 5 auf, von welchem sich etwa senkrecht nach hinten weg, auf das Gesicht des Benutzers zu ein Abstandshalteabschnitt 6 wegerstreckt, an dessen Rückseite längs der Oberkante ein Gesichtsanlagesteg 7 verläuft.

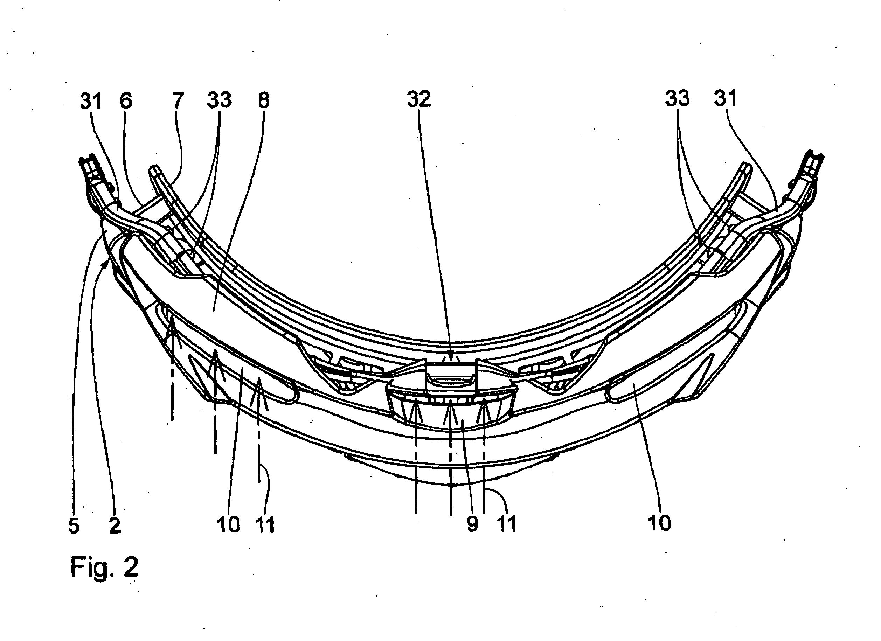

Oberhalb der Scheibe 3 ist an der Vorderseite des Rahmens 2 ein nach vorne über den Rahmen 2 vorstehender Schild 8 angeordnet, welcher eine zentrale Lufteinlassöffnung 9 und zwei seitliche Lufteinlassöffnungen 10 aufweist. Durch diese kann, wie durch die Pfeile 11 in Fig. 2 angedeutet, Luft einströmen, z.B. in Form von Fahrtwind oder aufgrund natürlicher äußerer Luftbewegungen. Der Schild 8 steht im Mittelbereich um mindestens 5mm, insbesondere mindestens 10 mm über die Sichtscheibe 3 der Skibrille 1 vor. Er bildet somit eine Luftleiteinrichtung, welche die auf die Skibrille 1 anströmende Luft gezielt lenkt und/oder umlenkt. Der Schild 8 ist vorzugsweise aus Kunststoff und/oder Karbon und/oder Metall, insbesondere Aluminium oder Titan.

Die Durcblassöffnungen 9, 10 sind mit Abstand von der Vorderkante 12 des Schildes 8 durch Aufwölbungen 13, 14 ausgebildet. Die Aufwölbungen 13, 14 weisen eine freie Höhe von mindestens 2 mm, insbesondere mindestens 5 mm auf. Sie weisen eine laterale Erstreckung von mindestens 10 mm, insbesondere mindestens 20 mm auf. Hierbei ist die laterale Erstreckung vorzugsweise mindestens dreimal, insbesondere mindestens fünfmal so groß die die freie Höhe der Aufwölbungen 13,14, was ihnen unter anderem ein besonders vorteilhaftes Design verleiht. Die Durchlassöffnungen 9, 10 sind derart ausgerichtet, dass beim Gebrauch der Skibrille 1 von vorne anströmende Luft effizient, das heißt weitgehend ungebremst durch sie durchströmen kann.

Der Rahmen 2 weist seinerseits an der Oberseite Durchlassöffnungen 15 im Mittelbereich und 16, 17 in den Randbereichen auf. Die Durchlassöffnungen 15, 16, 17 können durch luftdurchlässige Schaumstoffeinlagen 29 abgedeckt sein. Die Schaumstoffeinlagen 29 sind vorzugsweise auswechselbar und/oder waschbar. Zur Aufnahme und Fixierung der Schaumstoffeinlagen 29 sind spezielle Ausnehmungen 30 im Rahmen 2 vorgesehen. Im aufgesteckten Zustand bildet der Schild 8 eine Art Dach über den Durchlassöffnungen 15, 16, 17. Die Durchlassöffnungen 15, 16, 17 sind zumindest teilweise durch den Schild 8 abgedeckt. Sie sind somit bei aufgesetzter Brille von oben durch den Schild 8 gegen Eindringen von Regen und/oder Schnee geschützt.

Der Schild 8 ist so geformt und angeordnet, dass er im Mittelbereich relativ zu der Scheibe 3 von der Seite gesehen leicht nach unten geneigt ist. Der Schild 8 ist vorzugsweise auf den Rahmen 2 aufsteckbar. Er weist hiefür eine mittlere Steckeinrichtung 32 und jeweils seitliche Steckeinrichtungen 33 auf. Die Steckeinirichtungen 32, 33 sind mit dazu passenden Aufnahmen im Kopfbandbügel 31 verrastbar. Der Schild 8 ist somit bei Bedarf einfach abnehmbar. Im aufgesteckten Zustand ist der Schild 8 derart beabstandet zum Rahmen 2 angeordnet, dass zwischen diesen ein Lufteintrittsspalt 37 gebildet ist.

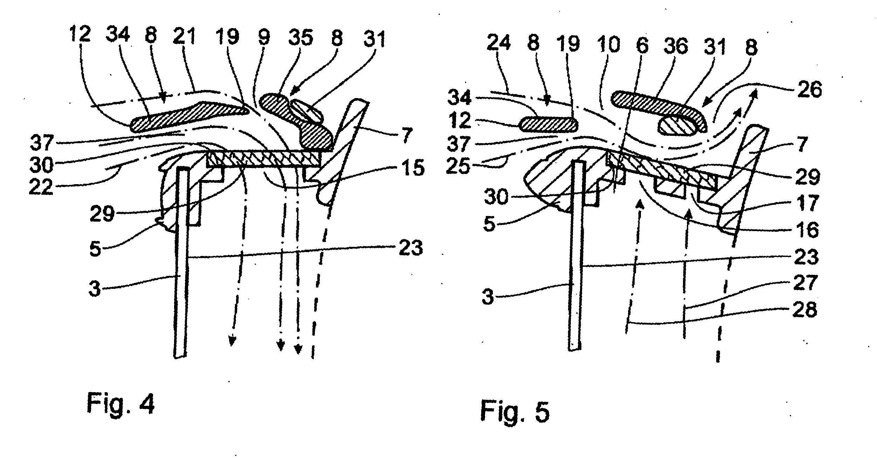

Die Ausgestaltung und Anordnung des Schildes 8 führt zu einer Luftströmung, wie sie in Fig. 4 und 5 dargestellt ist.

In Fig. 4 ist erkennbar, dass im Mittelbereich Luft (Luftströme 21, 22) oberhalb und unterhalb des Schildes 8 einströmt. Die Luft strömt somit durch die Lufteinlaßöffnung 9 und den Lufteintrittsspalt 37. Die anströmende Luft wird vom Schild 8 um ca. 90° umgelenkt, so dass sie längs der Hinterseite 23 der Scheibe 3 entlangströmt und so deren Beschlagen verhindert. Der Schild 8 weist hierzu im Mittelbereich einen vorderen Steg 34 auf, welcher tragflügelartig profiliert ausgebildet ist. Der Steg 34 weist eine flach zulaufende hintere Kante 19 auf, welche zu einer möglichst laminaren Strömung auf der dem Gesicht des Benutzers zugewandten Seite des Stegs 34 führt. Hierdurch werden Turbulenzen in diesem Bereich vermieden, was dazu fuhrt, dass die anströmende Luft besonders effizient an der Scheibe 3 entlangströmt. Durch die Vermeidung von Turbulenzen im Raum zwischen der Scheibe 3 und dem Gesicht des Benutzers wird außerdem die Luftzirkulation verbessert, wobei gleichzeitig eine ungünstige Luftströmung, welche zu erhöhter Tränenbildung in den Augen des Benutzers führt, vermieden wird.

Außerdem weist der Schild 8 einen die mittlere Aufwölbung 14 bildenden hinteren Steg 35 auf. Der hintere Steg 35 liegt am Gesichtsanlagesteg 7 an und verhindert so ein Ausweichen der Luftströmung nach oben. Er ist außerdem ausgehend vom Gesichtsanlagesteg 7 leicht nach vorne oben angestellt angeordnet, was zu einer besonders effizienten Umlenkung der anströmenden Luft beiträgt. Diese gezielte Umlenkung der anströmenden Luft kann durch eine leicht gebogene und/oder gestufte Ausbildung des hinteren Stegs 35 noch unterstützt werden. Durch den hinteren Steg 35 wird gewährleistetet, dass die anströmende Luft im Mittelbereich der Skibrille 1 zumindest weitestgehend in den Raum zwischen der Scheibe 3 und dem Gesicht des Benutzers gelenkt wird.

Der hintere Steg 35 ist mit dem Kopfbandbügel 31 verrastbar. Er ist insbesondere von unten mit dem Kopfbandbügel 31 verrastbar. Er wird dadurch sicher fixiert und trägt zum sicheren Halt des aufgesteckten Schildes 8 an der Skibrille 1 bei.

Im Folgenden wird unter Bezugnahme auf die Figur 5 die Luftströmung in den seitlichen Bereichen der Skibrille 1 beschrieben. Auch die seitlichen Aufwölbungen 13 sind jeweils von einem hinteren Steg 36 gebildet Aus Fig. 5 ist erkennbar, dass der hintere Steg 36 der seitlichen Aufwölbung 13 vom Gesichtsanlagesteg 7 beabstandet angeordnet ist. Zwischen dem hinteren Steg 36 und dem Gesichtsanlagesteg 7 ist ein Schlitz 26 ausgebildet. Die in den seitlichen Bereichen oberhalb und unterhalb des Schildes 8 eintretenden Luftströme 24, 25 können somit durch den Schlitz 26 vor dem Gesichtsanlagesteg 7 nach oben austreten.

Hierdurch wird aufgrund des Venturi-Effekts im Bereich hinter der Scheibe 3 ein Unterdruck erzeugt, so dass die dort befindliche feuchte und warme Luft, wie durch die Luftströme 27, 28 in Fig. 5 veranschaulicht, durch die Durchlassöffnungen 16,17 abgesogen wird, wodurch ebenfalls ein Beschlagen der Scheibe 3 verhindert wird. Vorzugsweise weist der hintere Steg 36 im Bereich der seitlichen Aufwölbungen 13 eine tragflügelartig profilerte Ausbildung auf. Eine derartige Ausbildung des hinteren Stegs 36 im Bereich der seitlichen Aufwölbungen 13 führt zu einem Unterdruck auf dessen Oberseite. Hierdurch wird der Venturi-Effekt in den seitlichen Bereichen der Skibrille 1 verstärkt und dadurch die Luftzirkulation im Raum zwischen der Scheibe 3 und dem Gesicht des Benutzers weiter verbessert.

Wie im Mittelbereich ist der hintere Steg 36 auch in den seitlichen Bereichen mit dem Kopfbandbügel 31 verrastbar. Im Gegensatz zum Mittelbereich ist er hier jedoch von oben mit dem Kopfbandbügel 31 verrastbar. Im Mittelbereich untergreift der hintere Steg 35 den Kopfbandbügel 31, wohingegen in den seitlichen Bereichen die hinteren Stege 36 den Kopfbandbügel 31 übergreifen. Dadurch ist zusammen mit den Steckeinrichtungen 32, 33 eine sehr stabile Rastverbindung zwischen dem Schild 8 und dem Rest der Skibrille 1 gegeben. Zugleich lässt sich diese Ratsverbindung aber ohne weiteres auch wieder lösbar.

Dadurch, dass der Schild 8 über die Scheibe 3 vorsteht, wirkt er gleichzeitig auch noch als Sonnenblende gegen von oben einfallende Sonneneinstrahlung und/oder als Regenschutz.

## Patentansprüche

1. Skibrille (1) umfassend einen Rahmen (2) und eine daran angeordnete Sichtscheibe (3) sowie ein Halteband (4) und Lufteinlassöffnungen (15, 16, 17) im Rahmen, **dadurch gekennzeichnet, dass** oberhalb der Scheibe (3) ein die Vorderseite der Scheibe (3) überragender, über die Scheibe (3) vorstehender Schild (8) angeordnet ist, welcher Lufteinlassöffnungen (9, 10) aufweist, wobei der Schild (8) im Abstand zum Rahmen (2) derart angeordnet ist, dass Luft durch die Lufteinlassöffnungen (9,_10) und unterhalb des Schildes (8) einströmen kann.

2. Skibrille (1) nach Anspruch 1, **dadurch gekennzeichnet, dass** im Mittelbereich die einströmende Luft durch Durchlassöffnungen (15) des Rahmens (2) in den Bereich zwischen der Hinterseite (23) der Scheibe (3) und der Stirn des Benutzers gelenkt wird.

3. Skibrille (1) nach Anspruch 1, **dadurch gekennzeichnet, dass** in den Randbereichen die durch Lufteinlassöffnungen (10) einströmende Luft (Luftströme 24, 25) oberhalb von Durchlassöffnungen (16, 17) im Rahmen (2) bzw. Rahmenbereich (6) zum Bereich hinter der Scheibe (3) entlanggeführt und durch Auslassöffnungen (26) an der Oberseite des Rahmens (2) wieder herausgeführt wird.

4. Skibrille (1) nach Anspruch 1, **dadurch gekennzeichnet, dass** der Schild (8) mit der Scheibe (3) einen Winkel von < als 90° einschließt.

5. Skibrille (1) gemäß einem der vorhergehenden Ansprüche, **dadurch gekennzeichnet, dass** ein Bügel (31) zur Befestigung eines Haltebands (4) vorgesehen ist.

6. Skibrille (1) gemäß Anspruch 5, **dadurch gekennzeichnet, dass** sich der Bügel (31) entlang der Oberkante der Skibrille (1) erstreckt.

7. Skibrille (1) gemäß einem der vorhergehenden Ansprüche, **dadurch gekennzeichnet, dass** der Schild (8) aufsteckbar ist.

8. Skibrille (1) gemäß einem der vorhergehenden Ansprüche, **dadurch gekennzeichnet, dass** der Schild aus Kunststoff und/oder Karbon und/oder einem Metall, insbesondere Aluminium oder Titan ist.

9. Skibrille (1) gemäß einem der vorhergehenden Ansprüche, **dadurch gekennzeichnet, dass** der Schild (8) mittels mindestens einer Steckeinrichtung (32, 33) mit der Skibrille (1) verrastbar ist.

10. Skibrille (1) gemäß einem der vorhergehenden Ansprüche, **dadurch gekennzeichnet, dass** der Schild (8) einen profilierten vorderen Steg (34) aufweist.

11. Skibrille (1) gemäß Anspruch 10, **dadurch gekennzeichnet, dass** der vordere Steg (34) eine flach zulaufende hintere Kante (19) aufweist.

12. Skbrille (1) gemäß einem der vorhergehenden Ansprüche, **dadurch gekennzeichnet, dass** der Schild (8) mindestens einen hinteren Steg (35, 36) aufweiset.

13. Skibrille (1) gemäß Anspruch 12, **dadurch gekennzeichnet, dass** der hintere Steg (35) in einem Mittelbereich an einem Gesichtsanlagesteg (7) anliegt.

14. Skibrille (1) gemäß Anspruch 12 oder 13, **dadurch gekennzeichnet, dass** der hintere Steg (36) in einem seitlichen Bereich von einem Gesichtsanlagesteg (7) beabstandet angeordnet ist.

## Claims

1. Ski goggles (1) comprising a frame (2) and a visibility pane (3) arranged thereon as well as a securing strap (4) and air inlet openings (15, 16, 17) in the frame, **characterised in that** above the pane (3) a shield (8) protruding over the front side of the pane (3) and projecting over the pane (3) is arranged, which comprises air inlet openings (9, 10), wherein the shield (8) is arranged at a distance from the frame (2) such that air can flow through the air inlet openings (9, 10) and underneath the shield (8).

2. Ski goggles (1) according to claim 1, **characterised in that** in the central area the inflowing air is guided through inlet openings (15) of the frame (2) into the area between the rear side (23) of the pane (3) and the forehead of the user.

3. Ski goggles (1) according to claim 1, **characterised in that** in the edge areas the air flowing in through air inlet openings (10) (air flows 24, 25) above through openings (16, 17) in the frame (2) or frame area (6) is guided along to the area behind the pane (3) and out again through outlet openings (26) on the upper side of the frame (2).

4. Ski goggles (1) according to claim 1, **characterised in that** the shield (8) encloses an angle of < 90° with the pane (3) .

5. Ski goggles (1) according to one of the preceding claims, **characterised in that** a curved frame (31) is provided for attaching a securing strap (4).

6. Ski goggles (1) according to claim 5, **characterised in that** the curved frame (31) extends along the upper edge of the ski goggles (1).

7. Ski goggles (1) according to one of the preceding claims, **characterised in that** the shield (8) can be fitted on.

8. Ski goggles (1) according to one of the preceding claims, **characterised in that** the shield is made of plastic and/or carbon and/or a metal, in particular aluminium or titanium.

9. Ski goggles (1) according to one of the preceding claims, **characterised in that** the shield (8) can be locked by means of at least one connecting device (32, 33) onto the ski goggles (1).

10. Ski goggles (1) according to one of the preceding claims, **characterised in that** the shield (8) has a profiled front segment (34).

11. Ski goggles (1) according to claim 10, **characterised in that** the front segment (34) has a flat tapered rear edge (19).

12. Ski goggles (1) according to one of the preceding claims, **characterised in that** the shield (8) comprises at least one rear segment (35, 36).

13. Ski goggles (1) according to claim 12, **characterised in that** the rear segment (35) bears in a central area against a face contacting segment (7).

14. Ski goggles (1) according to claim 12 or 13, **characterised in that** the rear segment (36) is arranged in a lateral area spaced apart from a face contacting segment (7).

## Revendications

1. Lunettes de ski (1) comprenant une monture (2) et un écran (3) qui y est agencé ainsi qu'un bandeau (4) et des ouvertures d'entrée d'air (15, 16, 17) dans la monture, **caractérisées en ce qu'**une visière (8) saillant de l'écran (3) et surplombant la face avant de l'écran (3) est agencée au-dessus de l'écran (3), laquelle visière présente des ouvertures d'entrée d'air (9, 10), la visière (8) étant agencée à distance de la monture (2) de telle sorte que l'air peut entrer par les ouvertures d'entrée d'air (9, 10) et sous la visière (8).

2. Lunettes de ski (1) selon la revendication 1, **caractérisées en ce que** dans la zone médiane, l'air entrant est dirigé par les ouvertures d'entrée d'air (15) de la monture (2) dans la zone située entre la face arrière (23) de l'écran (3) et le front de l'utilisateur.

3. Lunettes de ski (1) selon la revendication 1,
**caractérisées en ce que** dans les zones de bord, l'air entrant par des ouvertures d'entrée d'air (10) (flux d'air 24, 25) est conduit au-dessus des ouvertures de passage (16, 17) dans la monture (2) respectivement dans la zone de la monture (6) vers la zone derrière l'écran (3) et est à nouveau conduit vers l'extérieur par les ouvertures de sortie (26) sur la face supérieure de la monture (2).

4. Lunettes de ski (1) selon la revendication 1, **caractérisées en ce que** la visière (8) forme un angle < 90 avec l'écran (3).

5. Lunettes de ski (1) selon l'une quelconque des revendications précédentes, **caractérisées en ce qu'**un étrier (31) est prévu pour fixer un bandeau (4).

6. Lunettes de ski (1) selon la revendication 5, **caractérisées en ce que** l'étrier (31) s'étend le long de l'arête supérieure des lunettes de ski (1).

7. Lunettes de ski (1) selon l'une quelconque des revendications précédentes, **caractérisées en ce que** la visière (8) est enfichable.

8. Lunettes de ski (1) selon l'une quelconque des revendications précédentes, **caractérisées en ce que** la visière est en plastique et/ou carbone et/ou métal, en particulier de l'aluminium ou du titane.

9. Lunettes de ski (1) selon l'une quelconque des revendications précédentes, **caractérisées en ce que** la visière (8) peut s'enclencher avec les lunettes de ski (1) à l'aide d'au moins un dispositif d'enfichage (32, 33).

10. Lunettes de ski (1) selon l'une quelconque des revendications précédentes, **caractérisées en ce que** la visière (8) présente une aile avant (34) profilée.

11. Lunettes de ski (1) selon la revendication 10, **caractérisées en ce que** l'aile avant (34) présente une arête arrière (19) s'aplatissant.

12. Lunettes de ski (1) selon l'une quelconque des revendications précédentes, **caractérisées en ce que** la visière (8) présente au moins une aile arrière (35, 36).

13. Lunettes de ski (1) selon la revendication 12, **caractérisées en ce que** l'aile arrière (35) s'appuie dans une zone médiane contre une aile d'appui facial (7).

14. Lunettes de ski (1) selon la revendication 12 ou 13, **caractérisées en ce que** l'aile arrière (36) est agencée dans une zone latérale à distance d'une aile d'appui facial (7).
